# EUROPEAN PATENT SPECIFICATION

(11) **EP 2 009 441 B1**
(45) Date of publication and mention of the grant of the patent: **11.08.2010**
(21) Application number: 08159220.6
(22) Date of filing: 27.06.2008
(51) Int. Cl.: G01N 33/542

(54) **Method for determining the amount of DNA binding protein**
Verfahren zur Bestimmung einer DNA-Bindungsprotein-Menge
Procédé pour déterminer la quantité d'une protéine liant l'ADN

(30) Priority: 29.06.2007 JP 2007172216
(43) Date of publication of application: 31.12.2008
(73) Proprietor: SYSMEX CORPORATION, Kobe-shi, Hyogo 651-0073 (JP)
(72) Inventor: Koichi, Yamagata c/o Sysmex Corporation, Kobe-shi Hyogo 651-0073 (JP); Shigeki, Abe c/o Sysmex Corporation, Kobe-shi Hyogo 651-0073 (JP)
(74) Representative: De Clercq, Ann G. Y.

(56) References cited:
- US-A1- 2006 166 237
- KOBAYASHI T ET AL: "Detection of protein-DNA interactions in crude cellular extracts by fluorescence correlation spectroscopy" ANALYTICAL BIOCHEMISTRY, ACADEMIC PRESS INC. NEW YORK, vol. 332, no. 1, 1 September 2004 (2004-09-01), pages 58-66, XP004525465 ISSN: 0003-2697
- TSOURKAS ANDREW ET AL: "Hybridization kinetics and thermodynamics of molecular beacons" NUCLEIC ACIDS RESEARCH, OXFORD UNIVERSITY PRESS, SURREY, GB, vol. 31, no. 4, 15 February 2003 (2003-02-15), pages 1319-1330, XP002455592 ISSN: 0305-1048
- HAUSTEIN E ET AL: "Ultrasensitive investigations of biological systems by fluorescence correlation spectroscopy" METHODS : A COMPANION TO METHODS IN ENZYMOLOGY, ACADEMIC PRESS INC., NEW YORK, NY, US, vol. 29, no. 2, 1 February 2003 (2003-02-01), pages 153-166, XP002403400 ISSN: 1046-2023

### SEQUENCE LISTING

<110> Sysmex Corporation
<120> Measuring method of amount of DNA binding protein
<130> SYSM-022-EP
<150> JP 2007-172216
   <151> 29-06-2007
<160> 2
<170> PatentIn version 3.1
<210> 1
   <211> 48
   <212> DNA
   <213> Artificial
<220>
   <223> probe
<220>
   <221> stem_loop
   <222> (1)..(48)
   <223>
<400> 1
   agttgagggg actttcccag gcaaaagcct gggaaagtcc cctcaact 48
<210> 2
   <211> 46
   <212> DNA
   <213> Artificial
<220>
   <223> probe
<220>
   <221> stem_loop
   <222> (1)..(46)
   <223>
<400> 2
   caggcgcgtt ttgaccatct taaaaaagat ggtcaaaacg cgcctg 46

## Description

### FIELD OF THE INVENTION

The present invention relates to a method for determining the amount of a DNA binding protein.

### BACKGROUND

As a method of determining the amount of an objective DNA binding protein from a contaminant-containing sample such as a biological sample, there is a method disclosed in EP1138781. In this method, a complex having an obj ective DNA binding protein bound specifically to a probe is separated, and then the amount of the resulting complex is determined by an immunological quantification method or PCR.

An operation for separating the objective DNA binding protein/probe complex from a sample is troublesome. An operation for determining the amount of the objective DNAbinding protein and DNA contained in the complex by an immunological method or PCR is also troublesome. Accordingly, the method disclosed in EP1138781 takes some time until quantification results are obtained.

US2006166237 has proposed a method of detecting an objective DNA binding protein only in a short time by utilizing fluorescence correlation spectroscopy without an operation of separating and washing the DNA binding protein from a sample containing a plurality of proteins.

US2006166237 shows in the Examples that transcription factors such as AP-1 and NF-κB in a nuclear extract from a biological sample can be detected with fluorescent-labeled DNA probes. It is also shown therein that in the reaction of binding between a DNA binding probe used and an objective transcription factor, there is correlation between the concentration of the transcription factor and translational diffusion time to be measured. Further, correlation between addition of a stimulating factor (TNF-α) for activating a transcription factor (AP-1) and a labeled DNA probe-transcription factor complex is shown.

Fluorescence correlation spectroscopy is a method of measuring the translational diffusion time by the Brownian motion of molecules contained in a sample in a solution state. In US2006166237, a protein that specifically binds to a probe is detected by the prolongation of translational diffusion time by formation of a probe-protein complex. However, the Brownian motion of molecules in a sample in a solution state is liable to the influence of the viscosity of a solution. A biological sample contains a large amount of high-molecular materials such as contaminating proteins. It follows that even if the amount of an object sample-probe complex is the same in a biological sample, the diffusion time measured in the biological sample may change due to the influence of high-molecular substances contained in the solution sample.

EP1835283 discloses a method of supporting diagnosis of systemic inflammatory response syndrome (SIRS) including sepsis by measuring a transcription factor of inflammatory cytokine. In SIRS, pathological conditions change every hour. Accordingly, therapy to be used needs to depend on pathological conditions. Therefore, the amount of an object protein as a diagnostic material is determined desirably within 1 hour. Quantification methods requiring separation and washing of an objective protein can be applied to nonemergency protein quantification such as in medical checkup. However, such methods cannot be applied to diagnosis of a change in pathological conditions wherein the amount of an object protein contained in a sample collected from a patient should be determined in a short time. Accordingly, transcription factors should be measured in a short time by fluorescence correlation spectroscopy and the like.

Particularly in diagnosis of a change in pathological conditions, there has been demand for methods wherein the amount of transcription factors such as NF-κB and AP-1 that are DNA binding proteins is determined more accurately by fluorescence correlation spectroscopy, as described above.

### SUMMARY

The scope of the present invention is defined solely by the appended claims, and is not affected to any degree by the statements within this summary.

The object of the present invention is to provide a method for accurately determining the amount of an objective DNA binding protein contained in a contaminant-containing sample such as a biological sample by fluorescence correlation spectroscopy.

According to the quantification method of the present invention, the content of an objective DNA binding protein can be accurately quantified in a short time without troublesome operations such as separation and purification of the objective protein. Accordingly, the quantification method of the present invention can be applied to diagnosis of a change in pathological conditions.

### BRIEF DESCRIPTION OF THE DRAWINGS

FIG. 1 is a calibration curve showing the relationship between NF-κB and translational diffusion time.
FIG. 2 is a graph showing FCS measurement results by using a pure-line liquid sample.
FIG. 3 is a graph showing FCS measurement results of NF-κB in a nuclear extract sample.

### DETAILED DESCRIPTION OF THE EMBODIMENT

### Quantification Kit

First, the quantification kit used in the method for determining the amount of a DNA binding protein is described.

The kit for determining the amount of a DNAbinding protein is applied to measurement by fluorescence correlation spectroscopy. The quantification kit has a first measuring reagent and a second measuring reagent.

The first measuring reagent contains a fluorescent-labeled nucleic acid probe (Pfw) and a first unlabeled nucleic acid probe (Pw). An objective DNA binding protein can bind to a nucleic acid sequence of each of the fluorescent-labeled nucleic acid probe (Pfw) and the first unlabeled nucleic acid probe (Pw).

The second measuring reagent contains a fluorescent-labeled nucleic acid probe (Pfw) and a second unlabeled nucleic acid probe (Pn). An objective DNA binding protein does not bind to a nucleic acid sequence of the second unlabeled nucleic acid probe (Pn). That is, the second measuring reagent contains the fluorescent-labeled nucleic acid probe (Pfw) having a nucleic acid sequence that binds to the objective DNA binding protein and the unlabeled nucleic acid probe (Pn) having a nucleic acid sequence that does not bind to the objective DNA binding protein.

The term "DNAbinding protein" is a general name of proteins having affinity for a DNA and binding specifically or nonspecifically to the DNA. Examples of the DNA binding protein include a protein binding to a double-stranded DNA, a protein binding to a single-stranded DNA, a protein involved in maintaining a high-order structure of a chromosome, a DNA-dependent ATPase, and a DNA topoisomerase. The objective DNA binding protein to be measured in the present invention is preferably a DNA binding protein that binds specifically to a double-stranded DNA, more preferably a DNA binding protein that regulates gene expression. A more specific example of the objective DNA binding protein is a transcription factor. The transcription factor is preferably a transcription factor that regulates transcription of cytokine. The transcription factor of cytokine includes NF-κ, AP-1, NFAT, STAT family, IRF family, C/EBP, Sp1, and CREB. Among them, NF-κB is particularly preferable.

The nucleic acid sequence to which the objective DNA binding protein binds is not particularly limited as long as it contains a consensus sequence (Wseq) to which the objective DNAbinding protein binds. The nucleic acid sequence is suitably selected depending on the type of the objective DNA binding protein. For example, when the objective DNA binding protein is a transcription factor, a transcriptional regulatory domain involved in expression of the transcription factor, particularly an enhancer domain, can be selected as the nucleic acid sequence.

The fluorescent-labeled nucleic acid probe (Pfw) used in the present invention is a nucleic acid probe which is labeled with a fluorescent substance described later and which has a consensus sequence (Wseq) to which the objective DNA binding protein binds. The nucleic acid probe can be composed of DNA, RNA and PNA. As bases constituting the nucleic acid, adenine, guanine, cytosine, thymine, uracil and modified bases thereof can be used.

When the objective DNA binding protein binds to a double-stranded DNA, the fluorescent-labeled nucleic acid probe (Pfw) is preferably a nucleic acid probe that forms a stem-loop structure. This nucleic acid probe can be formed by allowing a sense chain and antisense chain of a double-stranded DNA containing a consensus sequence (Wseq) to be bridged via adenine or thymine forming a loop structure. The total number of bases in the fluorescent-labeled nucleic acid probe (Pfw) is not particularly limited, but is preferably 46 to 74.

The fluorescent substance used as a label is not particularly limited as long as it is a fluorescent substance capable of labeling the nucleic acid probe. Examples of such fluorescent substances include TAMRA, Rhodamine Green, Alexa 546, TMR, Alexa 488, and Alexa 647. Particularly, TAMRA and Rhodamine Green are preferable. These fluorescent substances are known in the art and are generally available.

The first unlabeled nucleic acid probe (Pw) used in the present invention is a nucleic acid probe which is not labeled with a fluorescent substance and which has a consensus sequence (Wseq) to which the objective DNA binding protein binds. It is desirable that the first unlabeled nucleic acid probe (Pw) and the fluorescent-labeled nucleic acid probe (Pfw) be the same nucleic acid sequence. However, their sequences other than the consensus sequence (Wseq) may be different in several bases from each other as long as their binding to the DNA binding protein is not influenced.

When the objective DNA binding protein binds to a double-stranded DNA, the first unlabeled nucleic acid probe (Pw) is also preferably a nucleic acid probe that forms a stem-loop structure similarly to the fluorescent-labeled nucleic acid probe (Pfw) described above.

The second unlabeled nucleic acid probe (Pn) used in the present invention is a nucleic acid probe which is not labeled with a florescent substance and which does not have a consensus sequence (Wseq) to which the objective DNA binding protein binds. The total number of bases in the second unlabeled nucleic acid probe (Pn) is preferably the same as in the first unlabeled nucleic acid probe (Pw). When the first unlabeled nucleic acid probe (Pw) forms a stem-loop structure, it is preferable that the second unlabeled nucleic acid probe (Pn) also forms a stem-loop structure.

The first measuring reagent in the quantification kit contains the fluorescent-labeled probe (Pfw) and the first unlabeled nucleic acid probe (Pw). In the first measuring reagent, the fluorescent-labeled probe (Pfw) is contained in such an amount that the objective DNA binding protein contained in a measurement sample can bind sufficiently to the fluorescent-labeled probe. In the first measuring reagent, the first unlabeled nucleic acid probe (Pw) is contained in a larger amount than the fluorescent-labeled probe (Pfw). The content of the first unlabeled nucleic acid probe (Pw) in the first measuring reagent is preferably 10-fold or more, more preferably 50-fold or more, based on the content of the fluorescent-labeled probe (Pfw).

Preferably, the first measuring reagent further contains poly (dIdC) to suppress nonspecific binding to the nucleic acid probe.

A solvent used for the first measuring reagent is a solvent wherein the nucleic acid probe and the objective DNA binding protein are stably present and can bind to each other. The solvent can be prepared appropriately depending on the obj ective DNA binding protein. Examples of such solvents include solutions having sodium chloride, EDTA and DTT added as necessary to buffers such as Tris buffer, HEPES buffer and phosphate buffer.

The second measuring reagent in the quantification kit contains the fluorescent-labeled probe (Pfw) and the second unlabeled nucleic acid probe (Pn). The second measuring reagent is the same as the first measuring reagent except that the second unlabeled nucleic acid probe (Pn) is contained in place of the first unlabeled nucleic acid probe (Pw). That is, the solvent for the second measuring reagent is the same as for the first measuring reagent. The second measuring reagent contains the fluorescent-labeled probe (Pfw) at the same concentration as in the first measuring reagent. The second measuring reagent contains the second unlabeled nucleic acid probe (Pn) in excess of Pfw.

The concentration of the first unlabeled nucleic acid probe (Pw) in the first measuring reagent and the concentration of the second unlabeled nucleic acid probe (Pn) in the second measuring reagent are preferably the same.

The measurement kit contains the first measuring reagent and the second measuring reagent. This measurement kit preferably contains instructions containing data showing the relationship between the objective DNA binding protein and translational diffusion time. The data are preferably in the form of a calibration curve showing the relationship between the amount of the objective DNA binding protein and translational diffusion time.

The measurement kit may contain a calculation program by which the amount of the objective DNA binding protein in a sample is calculated, on basis of the data described above, from the measured translational diffusion time. The calculation program can be provided via a recording medium recording it or via a phone line to the user of the measurement kit. The recording medium includes a flexible disk (FD), a compact disk (CD) and a magnetic recording disk (for example, DVD).

The calibration curve can be obtained by using a reaction solution for calibration containing substantially the fluorescent-labeled nucleic acid probe (Pfw) only and samples for calibration to which known amounts of the objective DNA binding protein were added. That is, two or more samples for calibration to which different amounts of the DNAbinding protein were added are prepared. Then, each sample for calibration is mixed with the reaction solution for calibration. Then, the translational diffusion time of each mixture is measured. From the relationship between the amount of the DNA binding protein in each of the samples for calibration and the measured translational diffusion time, a calibration curve is obtained. Preferably, a plurality of samples for calibration different in concentration are prepared by a serial dilution method. A line that shows correlation between the amount of the DNAbinding protein and the translational diffusion time in the respective samples for calibration is obtained by a least-squares method, whereby a calibration curve can be obtained.

The solvent used for the sample for calibration has the same basic composition as in the solvent for the first measuring reagent, but is preferably free of poly(dIdC).

### Quantification Method

Now, the method for determining the amount of a DNA binding protein according to the present invention is described.

The method for determining the amount of a DNA binding protein according to the present invention is a method for determining the amount of an objective DNA binding protein in a liquid sample, comprising steps of:
measuring a first translational diffusion time (Ta) of the floorescent-labeled substance present in a first mixture prepared by mixing the above-mentioned first measuring reagent and the above-mentioned liquid sample; wherein the first measuring reagent includes a fluorescent-labeled nucleic acid probe and a first unlabeled nucleic acid probe, and the target DNA binding protein is capable of binding to the fluorescent-labeled nucleic acid probe and the first unlabeled nucleic acid probe;
measuring a second translational diffusion time (Tb) of the floorescent-labeled substance present in a second mixture prepared by mixing the above-mentioned second measuring reagent and the above-mentioned liquid sample; wherein the second measuring reagent includes the fluorescent-labeled nucleic acid probe and a second unlabeled nucleic acid probe, and the target DNA binding protein is not capable of binding to the second unlabeled nucleic acid probe;
determining the amount of an objective DNAbinding protein contained in the above-mentioned liquid sample, based on the difference (|Ta - Tb|) between the first translational diffusion time (Ta) and the second translational diffusion time (Tb).

The liquid sample may be any liquid sample wherein the objective DNA binding protein is present in a free state without binding to a nucleic acid. The quantification method of the present invention can be adapted to a contaminant-containing liquid sample. Accordingly, the quantification method of the present invention can be preferably adapted to a biological sample collected from a patient.

The biological sample is not particularly limited as long as it is a sample containing cell components collected from a patient. The biological sample includes, for example, blood, urine, bronchioloalveolar lavage, saliva, sputum, brain bone marrow aspirate, synovial fluid, infiltrating fluid, and solubilized tissues.

There are transcription factors (for example, NF-κB and NFAT) that occur in an inactive state in cytoplasm, but upon activation, are localized in nuclei. Accordingly, when the amount of such a transcription factor in an activated state is tobedetermined, a nuclear extract is preferably used as a sample.

Biological samples including an intranuclear extract can be prepared by methods known in the art. For example, cells are swollen by treatment with a hypotonic solution, and cell membranes are destroyed with a homogenizer or a syringe. Then, a nuclear pellet is isolated by centrifugation. Then, the isolated nuclear pellet is treated with a hypertonic solution or a surfactant to extract nuclear proteins. Then, the resulting nuclear protein extract is centrifuged to recover a supernatant. This supernatant contains proteins extracted from nuclei.

The first measuring reagent in the quantification kit is mixed with the above liquid sample to prepare a first mixture. The mixing ratio is a ratio at which the objective DNA binding protein that can be contained in the liquid sample can sufficiently bind to the fluorescent-labeled probe (Pfw) contained in the first measuring reagent.

The prepared first mixture is left for 5 to 15 minutes, and then the first translational diffusion time (Ta) in the first mixture is measured. By leaving the first mixture for 5 to 15 minutes, a nucleic acid probe/DNA binding protein complex can be formed. This time can be established appropriately depending on the type and content of the objective DNA binding protein.

In the first mixture, the objective DNA binding protein contained in the liquid sample binds to the fluorescent-labeled probe (Pfw) and the first unlabeled nucleic acid probe (Pw) contained in the first measuring reagent. In the first measuring reagent, the unlabeled nucleic acid probe Pw is contained in excess of the fluorescent-labeled nucleic acid probe (Pfw). Accordingly, the objective DNA binding protein binds predominantly to Pw. Therefore, it is estimated that the first mixture contains the fluorescent-labeled nucleic acid probe (Pfw), an unlabeled nucleic acid probe Pw-objective DNA binding protein complex (Pw-protein complex), and the unlabeled nucleic acid probe (Pw). That is, it is estimated that the fluorescent-labeled substance in the first mixture is the fluorescent-labeled nucleic acid probe (Pfw).

The second measuring reagent in the measuring reagent kit is mixed with the above liquid sample, to prepare a second mixture. The mixing ratio of the second measuring reagent to the liquid sample is the same as the above-mentioned mixing ratio of the first mixture to the liquid sample.

The second translational diffusion time (Tb) in the prepared second mixture is measured. The time for which the second mixture is left is the same as the time for which the first mixture is left.

In the second mixture, the objective DNA binding protein contained in the liquid sample only binds to the fluorescent-labeled probe (Pfw) contained in the second measuring reagent. This is because the second unlabeled nucleic acid probe (Pn) contained in excess in the second measuring reagent does not substantially bind to the objective DNA binding protein. Accordingly, the fluorescent-labeled substance contained in the second mixture is assumed to be a fluorescent-labeled nucleic acid probe (Pfw) -target DNAbinding protein complex (Pfw-objective protein complex) and the unreacted fluorescent-labeled nucleic acid probe Pfw.

The first translational diffusion time (Ta) and the second translational diffusion time (Tb) can be determined by fluorescence correlation spectroscopy (FCS) by irradiating the first mixture and second mixtures respectively with a confocal laser light.

On the basis of the difference (|Ta - Tb|) between the first translational diffusion time (Ta) and the second translational diffusion time (Tb) thus determined, the amount of the objective DNA binding protein contained in the liquid sample is determined.

The fluorescent-labeled substance in the first mixture can be regarded as the nucleic acid probe (Pfw) not forming a complex. Accordingly, the first translational diffusion time (Ta) in the first mixture can be regarded as the translational diffusion time of Pfw. On the other hand, the fluorescent-labeled substance in the second mixture can be regarded as the Pfw-objective protein complex and the unreacted fluorescent-labeled nucleic acid probe Pfw. Accordingly, the second translational diffusion time (Tb) in the second mixture can be regarded as the translational diffusion time of the Pfw-objective protein complex and the unreacted fluorescent-labeled nucleic acid probe Pfw. Therefore, the difference (|Ta - Tb|) between the first translational diffusion time (Ta) and the second translational diffusion time (Tb) is considered to correspond to the difference in translational diffusion time based on the presence or absence of the Pfw-objective protein complex.

The amount of the objective DNA binding protein contained in the liquid sample can be determined from the data showing the relationship between the amount of the objective DNA binding protein and the translational diffusion time and from the difference (|Ta - Tb|) between the first translational diffusion time (Ta) and the second translational diffusion time (Tb). For example, when the data are in the form of a calibration curve showing the correlation between the translational diffusion time and target protein, the slope of the curve is determined and the difference in translational diffusion time is divided by the slope, whereby the amount of the protein can be calculated.

As used herein, fluorescence correlation spectroscopy (FCS) is a method of measuring the fluorescence emitted by molecules in solution when passing through a confocal region. Molecules in solution move freely by Brownian motion. Small molecules move rapidly and thus pass rapidly through the confocal region. Accordingly, the signal fluorescence intensity of small molecules rapidly changes. On the other hand, large molecules move slowly and thus pass slowly through the confocal region. Accordingly, the signal fluorescence intensity of large molecules slowly changes. FCS is a method wherein the movement velocity (translational diffusion time) of molecules is determined from the fluctuation rate of this signal fluorescence intensity by an autocorrelation method.

The assay system utilizing FCS is influenced by the viscosity of a solution. This is because the viscosity of a solution influences the Brownian motion of molecules. That is, the translational diffusion time may be prolonged by the factor other than molecular weight. More specifically, the translational diffusion time in a liquid sample with less contaminants and the translational diffusion time in abiological liquid sample are subtly different from each other even if the liquid sample and the biological sample contain the same amount of the Pfw-objective protein complex. The viscosity of a biological sample is generally high because it contains a large amount of high-molecular compounds such as proteins. Accordingly, the motion of the complex in the biological sample is inhibited, so the translational diffusion time tends to be prolonged.

In the quantification method of the present invention, however, the inhibitory condition, in the first mixture, of the Brownian motion of the Pfw-objective protein complex is the same as that in the second mixture. Accordingly, the prolongation of translational diffusion time attributable to the influence of contaminants contained in the liquid sample has been canceled in the difference (|Ta - Tb|) between the first translational diffusion time (Ta) in the first mixture and the second translational diffusion time (Tb) in the second mixture. Accordingly, the amount of the objective DNA binding protein contained in the liquid sample can be accurately determined on the basis of the difference (|Ta - Tb|).

As described above, the quantification method of the present invention can cancel the prolongation of translational diffusion time attributable to the influence of contaminants contained in a liquid sample. Accordingly, the amount of the objective DNA binding protein even in a biological sample can be accurately determined.

In the quantification method of the present invention, the first translational diffusion time (Ta) and the second translational diffusion time (Tb) can be measured within 30 minutes. Accordingly, the amount of the objective DNA binding protein can be determined within 30 minutes by using the measuring reagent kit provided with the calculation program described above.

### EXAMPLES

### Nucleic Acid Probe

### (1) Nucleic acid probe(Pfw) having a fluorescent-labeled consensus sequence (Wseq)

This nucleic acid probe (Pfw) is prepared by binding a fluorescent dye TAMRA (Sigma Genosys) to the 5'-terminus of a nucleic acidhaving the following nucleotide sequence that forms a stem-loop structure with 4 adenine bases as a loop. In the following sequence, Wseq is a region of from the 8- to 17-positions counted from the 5'-terminal.

### [Formula 1]

5' TAMRA-agttgaggggactttcccaggcaaaagcctgggaaagtcccctcaact 3'

As Pfw, an oligonucleotide (lyophilized product) synthesized by Sigma Genosys was dissolved at a Pfw concentration of 100 µM in 10 mM Tris-HCl buffer, then kept at 95°C for 10 minutes and at 65°C for 30 minutes to make it single-stranded, then returned again to room temperature to form a stem-loop structure and subjected to use.

### (2) Nucleic acid probe (Pw) having an unlabeled binding sequence (Wseq)

This nucleic acid probe (Pw) has a nucleotide sequence (sequence shown below) which is the same as that of Pfw except that the fluorescent dye is not bound thereto.

### [Formula 2]

5' agttgaggggactttcccaggcaaaagcctgggaaagtcccctcaact 3'

Like Pfw, Pw was made once single-stranded by heat denaturation, then returned again to room temperature to form a stem-loop structure and subjected to use.

### (3) Unlabeled and non-binding, unspecific type nucleic acid probe (Pn)

This nucleic acid probe (Pn) has the following nucleotide sequence forming a stem loop structure with 4 adenine bases as a loop.

### [Formula 3]

5' caggcgcgttttgaccatcttaaaacagatggtcaaaacgcgcctg 3'

Like Pfw, Pn was also made once single-stranded by heat denaturation, then returned again to room temperature to form a stem-loop structure and subjected to use.

### Reagents and Samples

### (1) FCS buffer

The FCS buffer is a solvent used for preparation of FCS measurement solutions and has the following composition.

**Table 1**

| | Blending quantity (µl) |
|---|---|
| 1M Tris (pH7.5) | 50 |
| 1M NaCl | 250 |
| 0.5M EDTA | 2 |
| 0.1M DTT | 25 |
| Glycerol | 100 |
| Water | 573 |

### (2) Recombinant NF-κB

A commercial product of Promega Corporation was used after dilution with a predetermined diluent. The diluent used was a solution having the following composition: 50 mM CaCl, 5 mM DTT, 20 mM HEPES (pH 7.9), 0.1% NP-40, and 10% glycol.

### (3) Nuclear extract of peripheral blood mononuclear cells from collected blood

Blood collected in a vacuum blood collection tube was diluted twice by adding an equal amount of physiological saline, and then layered on Ficoll (Amersham Bioscience) as a solution of specific gravity for lymphocyte isolation and centrifuged thereby separating it into erythrocytes, peripheral blood mononuclear cells and plasma components. The peripheral blood mononuclear cell fraction was recovered, then washed with physiological saline and centrifuged thereby removing the supernatant, to give a cell pellet.

For preparing a nuclear extract from the cell pellet, NucBuster (Trademark) that is a protein extraction kit of Novagen was used. That is, the cell pellet was dispersed in 75 µl of Reagent 1, then homogenized with Vortex for 15 seconds, and left on ice for 5 minutes. Thereafter, the sample was homogenized again with Vortex for 15 seconds and centrifuged at 15000 rpm at 4°C for 5 minutes to recover a supernatant as a cytoplasmic fraction. 40 µl of a mixture consisting of DTT, a protease inhibitor and Reagent 2 (mixing ratio 1 : 1 : 75) was added to the resulting pellet, and the mixture was homogenized with Vortex for 15 seconds and left on ice for 5 minutes. The sample was homogenized again with Vortex for 15 seconds and centrifuged at 15000 rpm at 4°C for 5 minutes, and the resulting supernatant was used as a nuclear extract.

### (4) Nuclear extract of HeLa cells

A culture of HeLa cells was centrifuged at 190 G for 5 minutes, and the recovered HeLa cells were washed with 1 ml ice-cold phosphate-buffered saline (PBS) to give a cell pellet. For preparing a nuclear extract from the cell pellet, NucBuster (Trademark) that is a protein extraction kit of Novagen was used. That is, the cell pellet was dispersed in 150 µl of Reagent 1, then homogenized with Vortex for 15 seconds, and left on ice for 5 minutes. Thereafter, the sample was homogenized again with Vortex for 15 seconds and centrifuged at 15000 rpm at 4°C for 5 minutes to recover a supernatant as a cytoplasmic fraction. 80 µl of a mixture consisting of DTT, a protease inhibitor and Reagent 2 (mixing ratio 1: 1: 75) was added to the resulting pellet, and the mixture was homogeni zed with Vortex for 15 seconds and left on ice for 5 minutes. The sample was homogenized again with Vortex for 15 seconds and centrifuged at 15000 rpm at 4°C for 5 minutes, and the resulting supernatant was used as a nuclear extract.

### Preparation of a calibration curve

1 µl of recombinant NF-κB (Promega) was mixed with 39 µl of a diluent (50 mM NaCl, 5 mM DTT, 20 mM HEPES (pH 7.9), 0.1% NP-40, 10% glycerol) to prepare a calibrator stock solution containing recombinant NF-κB at a concentration of 6 ng/µl.

4 µl of FCS buffer, 0.2 µl of 100 nM fluorescent-labeled nucleic acid probe Pfw (final concentration 1 nM), 8.8 µl of water, and 5 µl of Reagent 2 were mixed with one another to prepare a reaction solution for calibration.

Dilutions of the calibrator stock solution were mixed with the reaction solution for calibration, to prepare calibrators containing recombinant NF-κB at concentrations of 1.5 ng/µl, 0.8 ng/µl and 0.2 ng/µl, respectively. The respective samples were reacted at room temperature for 10 minutes, and then the translational diffusion time of each calibrator was measured with MF20 (Olympus). A mixture of a dilution containing recombinant NF-κB at a concentration of 0 ng/µl and the reaction solution for calibration was also measured for its translational diffusion time in the same manner as for the above calibrators.

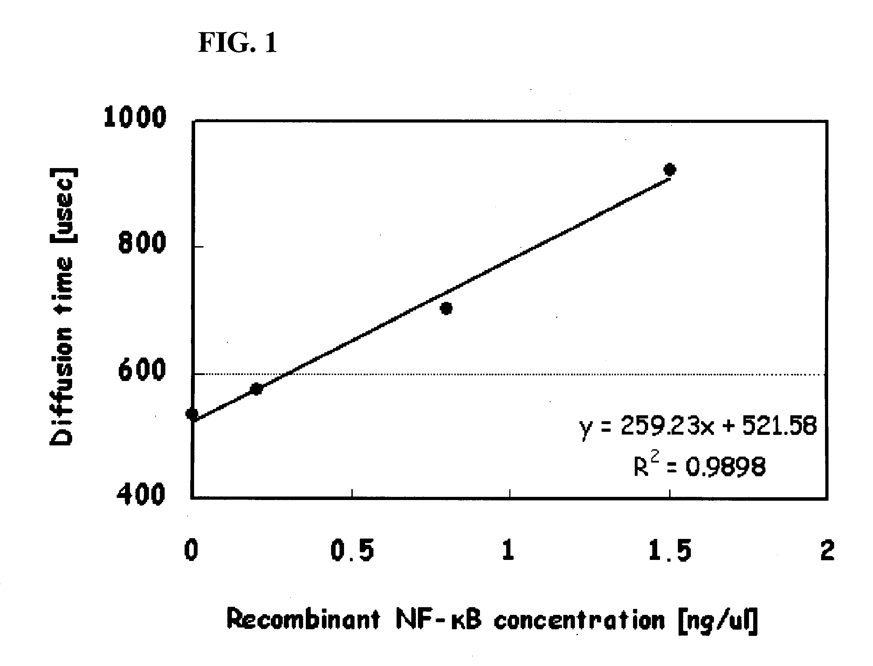

The concentration of recombinant NF-κB was plotted on the X-axis and the translational diffusion time on the Y-axis, to prepare a calibration curve by the least-squares method in the concentration range where linearity can be obtained. The obtained calibration curve is shown in FIG. 1.

### Translational diffusion time of a protein-probe complex in a pure-line liquid sample

### (1) Preparation of samples for FCS measurement

As a pure-line sample, 0.6 µg/µl solution of recombinant NF-κB was used. 5 µl of this pure-line liquid sample was mixed with FCS buffer, water and a nucleic acid probe, as shown in Table 2, to prepare Measurement Sample Nos. 1 to 5. As the control, a pure-line sample-free sample using the fluorescent-labeled probe Pfw only as a nucleic acid probe was used. In the Reference Example, the sample to which an anti-p50 antibody had further been added was used. The reaction time was 10 minutes for each sample.

**Table 2**

| Sample No. | 1 | 2 | 3 | 4 | 5 | Control | Reference Example |
|---|---|---|---|---|---|---|---|
| Recombinant NF-κB | 5 | 5 | 5 | 5 | 5 | - | 5 |
| FCS buffer | 4 | 4 | 4 | 4 | 4 | 4 | 4 |
| Recombinant diluent | 0 | 0 | 0 | 0 | 0 | 5 | 0 |
| Water | 10.8 | 10.6 | 9.8 | 10.6 | 9.8 | 10.8 | 10.8 |
| Pfw (100 nM) | 0.2 (1 nM) | 0.2 (1 nM) | 0.2 (1 nM) | 0.2 (1 nM) | 0.2 (1 nM) | 0.2 (1 nM) | 0.2 (1 nM) |
| Pw (1µM) | - | 0.2 (1 nM) | 1 (50 nM) | - | - | - | - |
| Pn (1µM) | - | - | - | 0.2 (1 nM) | 1 (50 nM) | - | - |
| Anti-p50 antibody (2 µg/µl) | - | - | - | - | - | - | 1 (0.1µl/µl) |

| | | | | | | | |
|---|---|---|---|---|---|---|---|
| Every content is expressed in µl. The concentration of each sample in round brackets is a final concentration. | | | | | | | |

### (2) FCS measurement

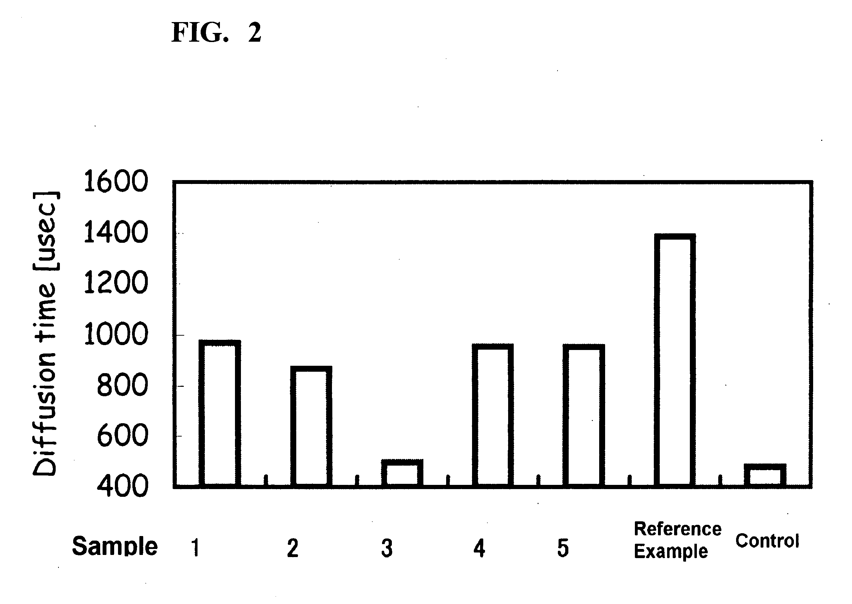

Sample Nos. 1 to 5, the control, and the reference sample (Reference Example) prepared above were measured for their translational diffusion time with MF20 manufactured by Olympus.
The results are shown in FIG. 2.

As compared with the control showing the translational diffusion time (478.2 µsec) based on the fluorescent-labeled probe Pfw, the translational diffusion time (969.3 µsec) of Sample No. 1 was longer due to formation of a fluorescent-labeled probe Pfw-NF-κB complex (abbreviated as Pfw-NF complex). Possibly because Sample 2 contains Pw acting as a competitive probe to reduce the degree of formation of the Pfw-NF complex, the translational diffusion time (868 µsec) of Sample 2 is shorter than that of Sample No. 1, but is longer than that of the control. The diffusion time (495.8 µsec) of Sample 3, wherein the amount of Pw is 50-fold, is almost the same as that of the control, and it can be said that in this sample, a majority of NF-κB formed a complex with Pw so that the fluorescent substance Pfw occurred in a state not forming a complex.

Samples 4 and 5 contain the nucleic acid probe to which NF-κB does not bind, and both of their translational diffusion times were the same degree as that of Sample 1. As can be seen from this result, the fluorescent substance in Samples 4 and 5 was the Pfw-NF complex.

An estimated reason that the diffusion time in the Reference Example was prolonged is that because the molecular weight of the anti-p50 antibody is larger than the molecular weight of the diffusion probe Pfw, the complex of the antibody and NF-κB is larger than the Pfw-NF complex, resulting in prolongation of the diffusion time.

Translational diffusion time of a complex between a probe and a protein from a nuclear extract of HeLa cells

### (1) Preparation of samples for FCS measurement

As a biological sample containing contaminants, a nuclear extract of HeLa cells in a cell strain of human cervical cancer was used to determine the amount of activated NF-κB (NF-κB transferred into nuclei) as a DNA binding protein. For obtaining an activated form of NF-κB from a state in cultured cells, that is, for transporting NF-κB into nuclei, TNF-α was added to a medium of HeLa cells, and then a nuclear extract was prepared, and as shown in Table 3, the nuclear extract, a solvent for FCS measurement, poly(dIdC), and a nucleic acid probe were mixed to prepare Samples 10 to 13. The reaction time was 5 minutes.

Sample 10 was a nuclear extract prepared without adding TNF-α. In the control, the nuclear extract was not added.

**Table 3**

| Sample No. | 10 | 11 | 12 | 13 | Control |
|---|---|---|---|---|---|
| TNF-α | - | + | + | + | - |
| Nuclear extract (6 mg/ml) | 5 | 5 | 5 | 5 | - |
| Reagent 2 | - | - | - | - | 5 |
| FCS buffer | 4 | 4 | 4 | 4 | 4 |
| Water | 10 | 10 | 10 | 10 | 10 |
| Poly (dIdC) (1.3 µg/µl) | 0.8 | 0.8 | 0.8 | 0.8 | 0.8 |
| Pfw (100 nM) | 0.2 | 0 | 0.2 | 0.2 | 0.2 |
| | (1 nM) | | (1 nM) | (1 nM) | (1 nM) |
| Pw (1µM) | 0 | 0 | 1 (50 nM) | 0 | 0 |
| Pn (1µM) | 0 | 0 | 0 | 1 (50 nM) | 0 |

| | | | | | |
|---|---|---|---|---|---|
| In the table, the content of each sample is expressed in µl, and a numerical value in round brackets shows a final concentration. In the items TNF-α and the nuclear extract, "+" indicates that the corresponding component was added, and "-" indicate that the component was not added. | | | | | |

### (2) FCS measurement

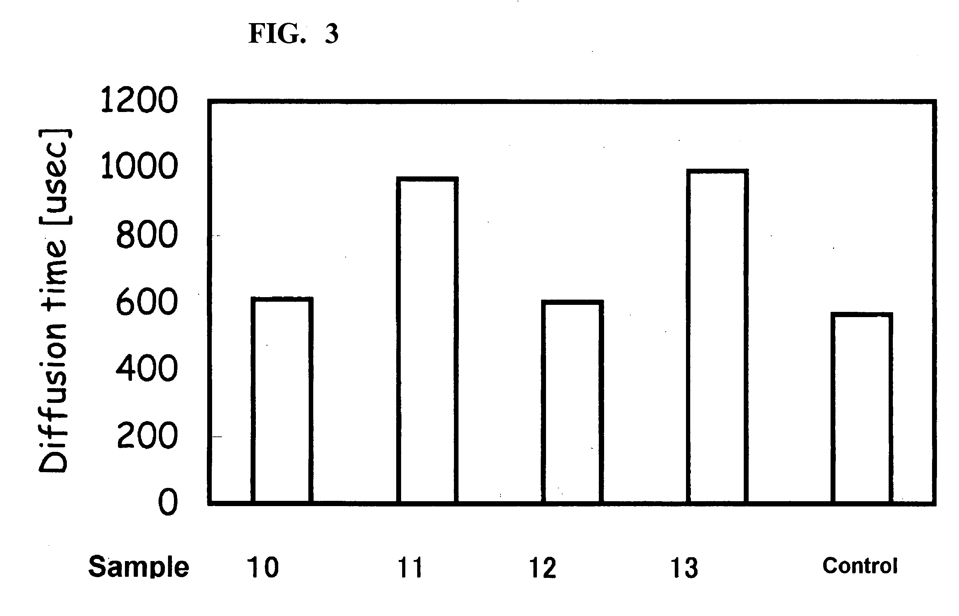

Samples 10 to 13 and the control were measured for their translational diffusion time with MF20 manufactured by Olympus. The results are shown in FIG. 3.

The difference between Sample 10 and Sample 11 is the presence or absence of simulation for converting NF-κB into activated NF-κB. The translational diffusion time of Sample 11 to which TNF-α was added is longer than that of Sample 10 to which TNF-α was not added, and it can be confirmed that the fluorescent-labeled probe Pfw has bound to NF-κB to form a complex (Pfw-NF complex). It can be seen that addition of TNF-α is necessary for transporting NF-κB into nuclei.

Inprinciple, activated NF-κB is considered seldom or never contained in Sample 10, and thus the fluorescent substance in this sample is considered to be the fluorescent-labeled probe Pfw in a free state, but the translational diffusion time (610.8 µsec) of Sample 10 was longer than the translational diffusion time (566.7 µsec) of the control. This is possibly because in Sample 10, a small amount of NF-κB is contained in nuclei even without stimulation with TNF-α or because the solution sample in Sample 10 has increased viscosity as compared with the nuclear extract-free control, thereby slightly inhibiting the degree of freedom of the Brownian motion of the fluorescent-labeled probe Pfw.

It is considered that by the coexistence of the competitive probe, a majority of NF-κB had formed a complex with the unlabeled probe Pw in Sample 12, so the fluorescent substance in this sample is the fluorescent-labeled probe Pfw in a free form. Because the translational diffusion time of Sample 12 is almost equal to the translational diffusion time of Sample 10, NF-κB is considered seldom or never contained in Sample 10.

The translational diffusion time of Sample 13 was almost equal to the translational diffusion time of Sample 11. Because the coexisting unlabeled probe Pn in Sample 13 is a probe with which NF-κB does not form a complex, the fluorescent substance present in Sample 13 is considered to be the Pfw-NF complex. Accordingly, the prolongation in translational diffusion time by formation of the Pfw-NF complex can be known not only by the difference between the translational diffusion time of Sample 10 and the translational diffusion time of Sample 11 by the presence or absence of NF-κB, but also by the difference between the translational diffusion time of Sample 12 and the translational diffusion time of Sample 13 by the presence or absence of formation of the Pfw-NF complex due to the coexistence of the competitive probe. The difference between the translational diffusion time of the control and the translational diffusion time of Sample 10 is considered attributable to the slight influence of an increase in solution viscosity on the degree of freedom of the Brownian motion of the fluorescent-labeled probe Pfw.

### Quantification of NF-κB in a nuclear extract: Verification by an addition/recovery experiment

### (1) Preparation of FCS measurement samples and measurement by FCS

Recombinant NF-κB was added at a concentration of 0.4 ng/µl to 10 µg of a nuclear extract prepared from a human monocyte lineage cell line U937 and then mixed with nucleic acid probes (Pfw, Pw, Pn), FCS buffer, Reagent 2, poly(dIdC) and water, as shown in Table 4, to prepare Measurement Samples 21, 22 and 23. Measurement Samples 21, 22 and 23 thus prepared were measured 3 times for the translational diffusion time of the fluorescent substance by FCS, to determine average translational diffusion time. The composition of each measurement sample and average translational diffusion time are shown in Table 4.

**Table 4**

| Sample No. | 21 | 22 | 23 |
|---|---|---|---|
| Recombinant NF-κB (0.4 ng/µl) | 2 | 2 | 2 |
| Nuclear extract (10 mg/ml) | 1 | 1 | 1 |
| Reagent 2 | 4 | 4 | 4 |
| FCS buffer | 4 | 4 | 4 |
| Water | 7.5 | 6.5 | 6.5 |
| Poly (dIdC) (1.3 µg/µl) | 1.3 | 1.3 | 1.3 |
| Pfw (100 nM) | 0.2 (1 nM) | 0.2 (1 nM) | 0.2 (1 nM) |
| Pw (1µM) | - | 1 (50 nM) | - |
| Pn (1µM) | - | - | 1 (50 nM) |
| Average translational diffusion time (µsec) | 650.7 | 547.4 | 655.9 |

| | | | |
|---|---|---|---|
| The content of each sample in the table is expressed in µl, and a numerical value in round brackets is a final concentration. | | | |

### (2) Calculation of the content and verification

On the basis of the calibration curve shown in FIG. 1, the amount of NF-κB was calculated from translational diffusion time.

The amount of NF-κB calculated from the difference between the translational diffusion time (650.7 µsec) of Sample 21 and the translational diffusion time (533 µsec) of the control wherein the concentration of NF-κB used in preparing the calibration curve is 0 ng/µl was 0.50 ng/µl. This corresponds to 124% recovery based on the amount of the NF-κB added to the sample. The amount of NF-κB calculated from the difference between the translational diffusion time of Sample 22 and the translational diffusion time of Sample 23 was 0.42 ng/µl. This corresponds to 105% recovery based on the amount of the NF-κB added to the sample.

Accordingly, the quantification method of the present invention utilizing a competitive assay system of unlabeled nucleic acid probes (Pw, Pn) could be confirmed to be a highly accurate quantification method wherein the content of a DNA binding protein calculated from a calibration curve is made highly reliable by accurately knowing the prolongation of translational diffusion time based on formation of a complex with a fluorescent-labeled nucleic acid probe, by cancelling the influence of contaminants contained in a sample.

The foregoing detailed description and examples have been provided by way of explanation and illustration, and are not intended to limit the scope of the appended claims.

## Claims

1. A method for determining the quantity of a target DNA binding protein in a liquid sample, comprising steps of:
measuring a first translational diffusion time of the fluorescent-labeled substance present in a first mixture prepared by mixing a first measuring reagent and the liquid sample, wherein the first measuring reagent includes a fluorescent-labeled nucleic acid probe and a first unlabeled nucleic acid probe, and the target DNA binding protein is capable of binding to the fluorescent-labeled nucleic acid probe and the first unlabeled nucleic acid probe;
measuring a second translational diffusion time of the fluorescent-labeled substance present in a second mixture prepared by mixing a second measuring reagent and the liquid sample, wherein the second measuring reagent includes the fluorescent-labeled nucleic acid probe and a second unlabeled nucleic acid probe, and the target DNA binding protein is not capable of binding to the second unlabeled nucleic acid probe; and
determining the quantity of the target DNA binding protein in the liquid sample based on a difference between the first translational diffusion time and the second translational diffusion time.

2. The method according to claim 1, wherein the determining step is performed so as to determine the quantity of the target DNA binding protein in a liquid sample based on the difference between the first translational diffusion time and the second translational diffusion time, and data representing a relationship between quantity of the target DNA binding protein and a translational diffusion time.

3. The method according to claim 2, wherein the data representing the relationship between quantity of the target DNA binding protein and the translational diffusion time is a calibration curve obtained by measuring a translational diffusion time in a mixture prepared by mixing a liquid solution including the fluorescent-labeled nucleic acid probe and a liquid solution including a predetermined quantity of the target DNA binding protein.

4. The method according to any one of claims 1 to 3, wherein
the amount of the first unlabeled nucleic acid probe included in the first measuring reagent is larger than the amount of the fluorescent-labeled nucleic acid probe included in the first measuring reagent, and
the amount of the second unlabeled nucleic acid probe included in the second measuring reagent is larger than the amount of the fluorescent-labeled nucleic acid probe included in the second measuring reagent.

5. The method according to any one of claims 1 to 4, wherein the concentration of the first unlabeled nucleic acid probe included in the first measuring reagent and the concentration of the second unlabeled nucleic acid probe included in the second measuring reagent are same.

6. The method according to any one of claims 1 to 5, wherein the liquid sample includes a nuclear extract.

7. The method according to any one of claims 1 to 6, wherein the target DNA binding protein is a transcription factor.

8. The method according to claim 7, wherein the transcription factor is a transcription factor of cytokine.

9. The method according to any one of claims 1 to 8, wherein the nucleic acid sequence of the fluorescent-labeled nucleic acid probe and the nucleic acid sequence of the first unlabeled nucleic acid probe are the same.

10. The method according to any one of claims 1 to 9, wherein at least one selected from the fluorescent-labeled nucleic acid probe, the first unlabeled nucleic acid probe and the second unlabeled nucleic acid probe forms a stem-loop structure.

## Patentansprüche

1. Verfahren zur Bestimmung der Menge eines DNA-bindenden Zielproteins in einer flüssigen Probe, umfassend die Schritte:
Messen einer ersten translationalen Diffusionszeit von der fluoreszenzmarkierten Substanz, die in einer ersten Mischung vorhanden ist, welche mittels Mischen eines ersten Messreagenzes und der flüssigen Probe hergestellt wurde, wobei das erste Messreagenz eine fluoreszenzmarkierte Nukleinsäuresonde und eine erste nicht markierte Nukleinsäuresonde enthält, und das DNAbindende Zielprotein in der Lage ist, an die fluoreszenzmarkierte Nukleinsäuresonde und an die erste nicht markierte Nukleinsäuresonde zu binden;
Messen einer zweiten translationalen Diffusionszeit von der fluoreszenzmarkierten Substanz, die in einer zweiten Mischung vorhanden ist, welche mittels Mischen eines zweiten Messreagenzes und der flüssigen Probe hergestellt wurde, wobei das zweite Messreagenz eine fluoreszenzmarkierte Nukleinsäuresonde und eine zweite nicht markierte Nukleinsäuresonde enthält, und das DNAbindende Zielprotein nicht in der Lage ist, an die zweite nicht markierte Nukleinsäuresonde zu binden; und
Bestimmen der Menge von dem DNA-bindenden Zielprotein in der flüssigen Probe auf der Grundlage einer Differenz zwischen der ersten translationalen Diffusionszeit und der zweiten translationalen Diffusionszeit.

2. Verfahren nach Anspruch 1, wobei der Bestimmungsschritt durchgeführt wird, um die Menge von dem DNA-bindenden Zielprotein in einer flüssigen Probe auf der Grundlage von der Differenz zwischen der ersten translationalen Diffusionszeit und der zweiten translationalen Diffusionszeit und von den Daten zu bestimmen, die einen Zusammenhang zwischen der Menge des DNA-bindenden Zielproteins und einer translationalen Diffusionszeit darstellen.

3. Verfahren nach Anspruch 2, wobei die Daten, welche den Zusammenhang zwischen der Menge von dem DNA-bindenden Zielprotein und der translationalen Diffusionszeit darstellen, eine Kalibrierungskurve sind, die durch Messen einer translationalen Diffusionszeit in einer Mischung erhalten wurde, die durch Mischen von einer flüssigen Lösung, welche die fluoreszenzmarkierte Nukleinsäuresonde enthält, und einer flüssigen Lösung, die eine vorbestimmte Menge von dem DNA-bindenden Zielprotein enthält, hergestellt wurde.

4. Verfahren nach einem der Ansprüche 1 bis 3, wobei
die Menge von der ersten nicht markierten Nukleinsäuresonde, die in dem ersten Messreagenz enthalten ist, größer ist als die Menge von der fluoreszenzmarkierten Nukleinsäuresonde, die in dem ersten Messreagenz enthalten ist; und
die Menge von der zweiten nicht markierten Nukleinsäuresonde, die in dem zweiten Messreagenz enthalten ist, größer ist als die Menge von der fluoreszenzmarkierten Nukleinsäuresonde, die in dem zweiten Messreagenz enthalten ist.

5. Verfahren nach einem der Ansprüche 1 bis 4, wobei die Konzentration von der ersten nicht markierten Nukleinsäuresonde, die in dem ersten Messreagenz enthalten ist, und die Konzentration von der zweiten nicht markierten Nukleinsäuresonde, die in dem zweiten Messreagenz enthalten ist, identisch ist.

6. Verfahren nach einem der Ansprüche 1 bis 5, wobei die flüssige Probe einen Kernextrakt enthält.

7. Verfahren nach einem der Ansprüche 1 bis 6, wobei das DNAbindende Zielprotein ein Transkriptionsfaktor ist.

8. Verfahren nach Anspruch 7, wobei der Transkriptionsfaktor ein Transkriptionsfaktor für Cytokine ist.

9. Verfahren nach einem der Ansprüche 1 bis 8, wobei die Nukleinsäuresequenz von der fluoreszenzmarkierten Nukleinsäuresonde und die Nukleinsäuresequenz von der ersten nicht markierten Nukleinsäuresonde identisch sind.

10. Verfahren nach einem der Ansprüche 1 bis 9, wobei mindestens eine Sonde, die ausgewählt ist aus der fluoreszenzmarkierten Nukleinsäuresonde, der ersten nicht markierten Nukleinsäuresonde und der zweiten nicht markierten Nukleinsäuresonde, eine Stamm-Schleifen-Struktur ausbildet.

## Revendications

1. Procédé de détermination de la quantité d'une protéine liant l'ADN cible dans un échantillon liquide, qui comprend les étapes consistant à :
mesurer un premier temps de diffusion translationnelle de la substance marquée par fluorescence présente dans un premier mélange préparé par mélange d'un premier réactif de mesure et de l'échantillon liquide, le premier réactif de mesure comprenant une sonde d'acide nucléique marquée par fluorescence et une première sonde d'acide nucléique non marquée, et la protéine liant l'ADN cible étant capable de se lier à la sonde d'acide nucléique marquée par fluorescence et à la première sonde d'acide nucléique non marquée ;
mesurer un second temps de diffusion translationnelle de la substance marquée par fluorescence présente dans un second mélange préparé par mélange d'un second réactif de mesure et de l'échantillon liquide, le second réactif de mesure comprenant la sonde d'acide nucléique marquée par fluorescence et une seconde sonde d'acide nucléique non marquée, et la protéine liant l'ADN cible n'étant pas capable de se lier à la seconde sonde d'acide nucléique non marquée ; et
déterminer la quantité de la protéine liant l'ADN cible dans l'échantillon liquide en se basant sur une différence entre le premier temps de diffusion translationnelle et le second temps de diffusion translationnelle.

2. Procédé selon la revendication 1, dans lequel l'étape de détermination est réalisée en vue de déterminer la quantité de la protéine liant l'ADN cible dans un échantillon liquide en se basant sur la différence entre le premier temps de diffusion translationnelle et le second temps de diffusion translationnelle, et les données représentant une relation entre la quantité de la protéine liant l'ADN cible et une durée de diffusion translationnelle.

3. Procédé selon la revendication 2, dans lequel les données représentant la relation entre la quantité de la protéine liant l'ADN cible et le temps de diffusion translationnelle sont une courbe d'étalonnage obtenue par mesure d'un temps de diffusion translationnelle dans un mélange préparé par mélange d'une solution liquide comprenant la sonde d'acide nucléique marquée par fluorescence et une solution liquide comprenant une quantité prédéterminée de la protéine liant l'ADN cible.

4. Procédé selon l'une quelconque des revendications 1 à 3, dans lequel
la quantité de la première sonde d'acide nucléique non marquée comprise dans le premier réactif de mesure est supérieure à la quantité de la sonde d'acide nucléique marquée par fluorescence comprise dans le premier réactif de mesure, et
la quantité de la seconde sonde d'acide nucléique non marquée comprise dans le second réactif de mesure est supérieure à la quantité de la sonde d'acide nucléique marquée par fluorescence comprise dans le second réactif de mesure.

5. Procédé selon l'une quelconque des revendications 1 à 4, dans lequel la concentration de la première sonde d'acide nucléique non marquée comprise dans le premier réactif de mesure et la concentration de la seconde sonde d'acide nucléique non marquée comprise dans le second réactif de mesure sont identiques.

6. Procédé selon l'une quelconque des revendications 1 à 5, dans lequel l'échantillon liquide comprend un extrait nucléaire.

7. Procédé selon l'une quelconque des revendications 1 à 6, dans lequel la protéine liant l'ADN cible est un facteur de transcription.

8. Procédé selon la revendication 7, dans lequel le facteur de transcription est un facteur de transcription des cytokines.

9. Procédé selon l'une quelconque des revendications 1 à 8, dans lequel la séquence d'acide nucléique de la sonde d'acide nucléique marquée par fluorescence et la séquence d'acide nucléique de la première sonde d'acide nucléique non marquée sont identiques.

10. Procédé selon l'une quelconque des revendications 1 à 9, dans lequel au moins l'une de la sonde d'acide nucléique marquée par fluorescence, de la première sonde d'acide nucléique non marquée et de la seconde sonde d'acide nucléique non marquée forment une structure tige-boucle.
